# EUROPÄISCHE PATENTSCHRIFT

(11) **EP 3 166 552 B1**
(45) Veröffentlichungstag und Bekanntmachung des Hinweises auf die Patenterteilung: **12.09.2018**
(21) Anmeldenummer: 15744138.7
(22) Anmeldetag: 13.07.2015
(51) Int. Cl.: A61F 9/02, G02C 7/10

(54) **VORRICHTUNG UND VERFAHREN ZUM SCHUTZ DER AUGEN VOR STRAHLUNG**
DEVICE AND METHOD FOR PROTECTING THE EYES FROM RADIATION
DISPOSITIF ET PROCÉDÉ DE PROTECTION DES YEUX CONTRE UN RAYONNEMENT

(30) Priorität: 13.07.2014 EP 14176838
(43) Veröffentlichungstag der Anmeldung: 17.05.2017
(73) Patentinhaber: Caruso, Giuseppe, 8212 Neuhausen am Rheinfall (CH)
(72) Erfinder: Caruso, Giuseppe, 8212 Neuhausen am Rheinfall (CH)
(86) Internationale Anmeldenummer: PCT/EP2015/065958
(87) Internationale Veröffentlichungsnummer: WO 2016/008839

(56) Entgegenhaltungen:
- EP-A1- 0 335 056
- FR-A1- 2 742 555
- GB-A- 2 152 661
- US-A- 5 255 117

## Beschreibung

Die Erfindung betrifft eine Vorrichtung zum Schutz der Augen vor Strahlung. Die Erfindung betrifft weiter ein Verfahren zum Schutz der Augen vor Strahlung

### Stand der Technik

Es finden immer mehr Attacken gegen Personen statt, bei welchen Laser, starke Lichtstrahlen oder Infrarot verwendet werden. Es ist daher ein Bedürfnis die Augen dieser Personen, beispielsweise Piloten, Busfahrer, Lokomotivführer, Polizisten oder Militär, besser vor solchen Strahlen zu schützen, um zum Beispiel gesundheitliche Schäden oder Unfälle zu vermeiden.

Es sind Vorrichtungen zum Schutz der Augen vor Strahlungsattacken bekannt, beispielsweise aus den Dokumenten US5828437 oder US5255117. Solche Vorrichtungen weisen den Nachteil auf, dass das Auge nur ungenügend geschützt ist.

### Darstellung der Erfindung

Aufgabe der Erfindung ist ein Vorrichtung sowie ein Verfahren zum Augenschutz der Personen.

Diese Aufgabe wird gelöst mit einer Vorrichtung aufweisend die Merkmale von Anspruch 1. Die abhängigen Ansprüche 2 bis 10 betreffen weitere vorteilhafte Ausgestaltungen. Die Aufgabe wird weiter gelöst mit einem Verfahren aufweisend die Merkmale von Anspruch 11. Die abhängigen Ansprüche 12 bis 15 betreffen weitere vorteilhafte Verfahrensschritte.

Die Aufgabe wird insbesondere gelöst mit einer Vorrichtung zum Schutz der Augen vor Strahlung, vorzugsweise im Bereich von 100nm bis 1 mm und insbesondere vor UV, Licht, oder IR, umfassend zumindest zwei Sensoranordnungen, eine äussere Sensoranordnung sowie eine innere Sensoranordnung, wobei jede Sensoranordnung eine Mehrzahl von nacheinander folgend angeordneten Strahlensensoren aufweist welche entlang je eines geschlossenen Kurvenzuges angeordnet sind, und wobei die innere Sensoranordnung von der äusseren Sensoranordnung umschlossen ist, und wobei die äussere und die innere Sensoranordnung aneinander liegend angeordnet sind, und wobei die innere Sensoranordnung einen Strahlendurchgangsbereich umschliesst, und wobei der Strahlendurchgangsbereich eine Strahlendurchgangsöffnung aufweist, und wobei eine Schliessvorrichtung derart bezüglich dem Strahlendurchgangsbereich angeordnet ist, dass eine durch die Strahlendurchgangsöffnung tretende Einfallsstrahlung entweder durchgelassen oder komplett geschlossen oder zumindest teilweise durch eine getönte Oberfläche abgeschwächt wird, sowie umfassend eine Ansteuervorrichtung welche mit der inneren Sensoranordnung, der äusseren Sensoranordnung sowie der Schliessvorrichtung verbunden ist.

Die Aufgabe wird weiter insbesondere gelöst mit einem Verfahren zum Schutz der Augen vor Strahlung, vorzugsweise im Bereich von 100nm bis 1 mm und insbesondere vor UV, Licht, oder IR, wobei das Auge von zumindest zwei Sensoranordnungen umgeben ist, einer äusseren Sensoranordnung sowie eine inneren Sensoranordnung, wobei jede Sensoranordnung eine Mehrzahl von nacheinander folgend angeordneten Strahlensensoren aufweist welche entlang je eines geschlossenen Kurvenzuges angeordnet sind, wobei die die innere Sensoranordnung von der äusseren Sensoranordnung umschlossen ist, und wobei die innere Sensoranordnung einen Strahlendurchgangsbereich umschliesst, und wobei eine Strahlendurchgangsöffnung im Strahlendurchgangsbereich angeordnet ist, und wobei eine Schliessvorrichtung derart angeordnet ist, dass eine durch die Strahlendurchgangsöffnung tretende Einfallsstrahlung entweder durchgelassen oder zumindest teilweise abgeschwächt wird, wobei die Schliessvorrichtung geschlossen wird, wenn die äussere Sensoranordnung und nachfolgend die innere Sensoranordnung von der Einfallsstrahlung bestrahlt wird, oder nur die zweite Sensoranordnung bestrahlt wird und wobei die Schliessvorrichtung wieder geöffnet wird, wenn vorerst die innere Sensoranordnung und nachfolgend nur noch die äussere Sensoranordnung von der Einfallsstrahlung bestrahlt wird und wobei beide Sensoranordnung miteinander bestrahlt werden, dann wird die Schliessvorrichtung geschlossen, sobald die programmierten Werte überschritten werden.

Unter dem Begriff "Licht" wird hierin sichtbares Licht in einem Wellenbereich von 380nm bis 780 nm verstanden.

Strahlungsattacken werden häufig mit Lasern durchgeführt. So werden beispielsweise Piloten durch Laserattacken geblendet. Laserstrahlen haben abhängig von der Distanz der Attacke eine unterschiedliche Punktgrösse beziehungsweise einen unterschiedlichen Strahlendurchmesser. Bei 10000m Distanz kann die Punktgrösse ca. 10m betragen, bei 2500m ca. 2.5m, bei, 1250 m ca. 1.25m haben. Bei näheren Distanzen wie 50m ca. 0.05m und bei 25m 0.025m und bei einer Distanz von 10m von nur 0.01m.

Die erfindungsgemässe Vorrichtung weist den Vorteil auf, dass auch eine kleine Punktgrösse beziehungsweise ein kleiner Strahlungsdurchmesser sicher ausgeblendet werden kann, weil die Vorrichtung das Annähern des Strahls erkennt und die Schliessvorrichtung den Strahl unterbricht, sobald dieser die äussere Sensoranordnung und nachfolgend die innere Sensoranordnung beleuchtet, sodass der Strahl nicht mehr auf das Auge fällt. Sobald der Strahl über die innere Sensoranordnung wieder zur äusseren Sensoranordnung gelangt ist, und die innere Sensoranordnung nicht mehr beleuchtet wird, kann die Schliessvorrichtung wieder geöffnet werden. Dies hat in der Praxis zur Folge, dass die Schliessvorrichtung nur über eine sehr kurze Zeit geschlossen ist, so dass das Auge des Piloten nur während einer sehr kurzen Zeit abgedeckt ist, und der Pilot während der meisten Zeit über eine freie Sicht verfügt ohne dazu abgelenkt zu werden. Als Schliessvorrichtung wird vorzugsweise eine mechanische Schliessvorrichtung oder aber auch eine elektronische Schliessvorrichtung verwendet, wie diese beispielsweise von Fotoapparaten bekannt sind, mit Verschlusszeiten von vorzugsweise im Bereich von 10⁻³ bis 10⁻¹⁸ Sekunden. Die Verschlusszeiten können somit sehr kurz sein, zum Beispiel im Bereich von Millisekunden bis Atto-Sekunden. Ein Augenschlag dauert etwa 0,25s. Das Auge ist somit zu träge um sich wirksam vor Laserattacken zu schützen. Unter Verschlusszeit wird die Zeitdauer verstanden, um die Schliessvorrichtung vom geöffneten Zustand in den geschlossenen Zustand überzuführen. Die kurze Verschlusszeit der erfindungsgemässen Vorrichtung weist somit den Vorteil auf, dass das Auge des Piloten oder betroffenen Person besser geschützt werden kann da das Augenlied sich nur in ca. 0.25 Sek. schliesst, insbesondere auch bei Laserattacken welche aus kurzer und weiter Distanz ausgeführt werden und eine höhere Leistung aufweisen. Grundsätzlich gilt, je stärker die Laserstrahlung, umso schneller sollte der Verschluss geschlossen werden, um das Auge vor einer übermässigen Strahlungsbelastung zu schützen, da die Grenzwerte je nach Strahlungsstärke (m/W) in Zeit gemessen kürzer werden. Die erfindungsgemässe Vorrichtung weist die besonders vorteilhafte Eigenschaft auf, dass der Verschluss bzw. die Schliessvorrichtung vorzugsweise sofort wieder geöffnet wird, und dies vorzugsweise sogar individuell, d.h. also rechte und linke Seite arbeiten autonom, sobald sich der Laserstrahl nicht mehr innerhalb des Strahlendurchgangsbereichs befindet, so dass der Pilot Lokomotivführer, Busfahrer usw. auf dem attackierten Auge sehr schnell wieder über eine freie Sicht verfügt. In einer vorteilhaften Ausgestaltung liegt die Öffnungszeit, das heisst die Zeitdauer um die Schliessvorrichtung vom geschlossenen Zustand in den geöffneten Zustand überzuführen, ebenfalls vorzugsweise im Bereich von 10⁻³ bis 10⁻¹⁸ Sekunden. Somit bleibt die Schliessvorrichtung vorzugsweise solange geschlossen, bis sich der Laserstrahl nicht mehr innerhalb des Strahlendurchgangsbereichs befindet, um danach sofort wieder geöffnet zu werden. Vorteilhafterweise bleibt die Schliessvorrichtung abhängig von der Bestrahlungsdauer, während welcher der Strahlendurchgangsbereich vom Laserstrahl bestrahlt wird, geschlossen, wobei die Bestrahlungsdauer je nach Situation im Bereich von Bruchteilen von Sekunden liegen kann, in ungünstigen Fällen mit längerer Bestrahlungsdauer jedoch auch beispielsweise im Bereich von etlichen Sekunden liegen kann. In einer besonders vorteilhaften Ausgestaltung ist vor dem linken und dem rechten Auge ein separater Verschluss angeordnet, wobei die beiden Verschlüsse unabhängig voneinander angesteuert werden. Dadurch ist es bei einer Laserattacke mit kleinen Laserstrahlgrössen möglich, dass nur entweder das linke oder das rechte Auge gleichzeitig attackiert wird, was zur Folge hat, dass üblicherweise zumindest ein Auge über eine freie Sicht verfügt.

Die erfindungsgemässe Vorrichtung weist in einer besonders vorteilhaften Ausgestaltung den Vorteil auf, dass die Bewegungsrichtung einer Strahlenattacke erkannt werden kann, weil die Vorrichtung zwei nebeneinander angeordnete Sensoranordnungen aufweist, wobei jede Sensoranordnung eine Mehrzahl von individuell messbaren Sensoren aufweist. Basieren auf diesen Messwerten ist es mithilfe einer Auswertevorrichtung möglich die Strahlstärke zu berechnen. In einer weiteren vorteilhaften Ausgestaltung wird die gemessene Einfallsrichtung in der erfindungsgemässen Vorrichtung gespeichert, vorzugsweise zusammen mit weiteren Daten wie die Uhrzeit, die Strahlungsintensität oder der Ort, Koordinaten beispielsweise basierend auf GPS Daten oder die Höhe, die zum Beispiel mit MEMS-Sensoren (CRG20, SiRRS01, PinPoint, Gemini) und Elektronik aufgenommen werden können und via Kommunikation Telefon, SMS, etc. weitergeleitet werden können. Basieren auf diesen Daten ist es vorzugsweise sofort oder nachträglich möglich den Emissionsort der Laserattacke zumindest ungefähr zu bestimmen.

Bei der erfindungsgemässen Vorrichtung wird vorteilhafterweise eine mechanische oder elektronische Schliessvorrichtung verwendet, wie diese beispielsweise bei Fotoapparaten bekannt ist. Solche Schliessvorrichtungen werden auch als Schutter bezeichnet. Eine derartige Schliessvorrichtung weisst im geöffneten Zustand üblicherweise eine Strahlentransmission von 100% oder kleiner auf, d.h. der durch die Schliessvorrichtung eintretende Strahl unterliegt keiner oder einer nur geringen Dämpfung.
Die erfindungsgemässe Vorrichtung ist daher besonders vorteilhaft für Piloten, da die Normen ISO12311-1, ISO12312-1 oder EN166 vorschreiben, dass eine Lichttransmission von 75% erforderlich ist.

Die erfindungsgemässe Vorrichtung weist somit auch den Vorteil auf, dass eine gute Farberkennung gewährleistet ist und keine Farbbeeinträchtigung stattfindet, da keine Wellenlängen weggefiltert werden. Somit erfüllt die erfindungsgemässe Vorrichtung zum Beispiel in der Fliegerei, Schifffahrt, Eisenbahn und in Verkehrsbereich vorgeschriebenen Normen ISO 12312-1, ISO12311-1, EN166 bezüglich Farberkennung, insbesondere von modernen farbigen digitalen oder analogen Displays.

Eine mechanische Schliessvorrichtung weist zudem den Vorteil auf, dass diese problemlos Strahlungen in einem grossen Wellenbereich unterbrechen kann. In der erfindungsgemässen Vorrichtung werden beispielsweise Sensoren verwendet mit einer Empfindlichkeit in Wellenbereich zwischen 400 nm bis 1100 nm.

Als Schutz vor Laserattacken sind beispielsweise Polarisationsfilter oder Laserschutzbrillen bekannt. Nachteilig an Polarisationsfiltern ist die Tatsache, dass diese in der Fliegerei nicht zugelassen sind, da die Bordinstrumente nicht mehr erkennbar sind. Nachteilig an Laserschutzbrillen ist die Tatsache, dass Laser mit einer Vielzahl unterschiedlicher Wellenlängen bekannt sind. Laserschutzbrillen können jedoch nur die fest eingebauten und vorgegebenen Wellenlängen filtern, sodass die Gefahr besteht, dass eine verwendete Laserschutzbrille den einstrahlenden Laser nicht filtert.

Die erfindungsgemässe Vorrichtung weist den weiteren Vorteil auf, dass auch Laserattacken mit hoher Strahlung stärke vollständig blockiert werden können, da der optische Strahlenpfad durch eine mechanische Schliessvorrichtung unterbrochen wird. Bekannte Vorrichtungen lassen ab einer gewissen Strahlungsstärke eine Reststrahlung durch, so dass kein vollständiger Schutz gewährleistet ist. Beispielsweise kann ein Punktstrahl eine LCD durchdringen und es kommt noch darauf an im welchem Winkel die Strahlung beim LCD hinein kommt, kann der Strahl stärker oder schwächer ausfallen. Beim bevorzugten Ausführungsbeispiel der vorliegenden Erfindung wird die Strahlendurchgangöffnung mit Hilfe der Schliessvorrichtung komplett geschlossen. Somit ist es nicht möglich, dass bei stärkeren Lasern das Restlicht trotzdem Schäden verursachen können.

Die erfindungsgemässe Vorrichtung kann in einer weiteren vorteilhaften Ausgestaltung zudem zumindest eine Kamera und zumindest ein Display aufweisen, wobei der Display im Innern der Brille angeordnet ist, und die Kamera aussen an der Brille angeordnet ist, so dass die Kamera Daten in 2D oder 3D oder in SWIR, MWIR, LWIR ein Aussenbild aufnimmt, und am Display wiedergibt, so dass einem Piloten die Umgebung so gut als möglich dargestellt werden kann, selbst wenn beide Schliessvorrichtungen kurzfristig geschlossen sind. Falls eine Schnittstelle des Computers besteht ist es auch möglich zusätzlich Daten wie Flugdaten direkt auf den Bildschirm anzuzeigen.

Die erfindungsgemässe Messevorrichtung kann in einer weiteren vorteilhaften Ausgestaltung zudem ein Filter umfassen, welches vor der Brille angeordnet wird, und die eingehende Strahlung wie UVC, UVB, UVA und Blaulicht und Infrarot filtert.

Die Erfindung wird nachfolgend anhand von Ausführungsbeispielen im Detail beschrieben.

### Kurze Beschreibung der Zeichnungen

Die zur Erläuterung der Ausführungsbeispiele verwendeten Zeichnungen zeigen:
- Fig. 1: eine perspektivische Ansicht einer als Brillenaufsatz ausgestalteten Vorrichtung.
- Fig. 2: eine Draufsicht auf eine Brille mit Brillenaufsatz;
- Fig. 3: eine schematische Ansicht einer Vorrichtung mit einer Schliessvorrichtung unter Verwendung von LCDs oder anderen Technologien die eine Verdunklung ermöglichen;
- Fig. 4a-4e: ein beispielhafter Ablauf zum Schliessen und Öffnen der Vorrichtung;
- Fig. 5: ein schematisches Signalschaltbild einer Ansteuervorrichtung.

Grundsätzlich sind in den Zeichnungen gleiche Teile mit gleichen Bezugszeichen versehen.

### Wege zur Ausführung der Erfindung

Die Schutzvorrichtung gegen Laserattacken, erhöhter Strahlung oder stärkeres Licht, die auf ein ausgeklügeltes UV, Licht- und IR Sensoren System basiert und den Impuls in milli-, micro-, nano-, pico-, femto-, atto-, zepto- bis zu yocto- Sekunden an den Verschluss weiter gibt und mindestens einen oder mehrere Verschlüsse in milli-, micro-, nano-, oder bis zu atto oder schneller schliessen und öffnen kann. Dieser Laser- und erhöhter Lichtschutz soll das Auge vor gefährlichen und intensiven Strahlen, die Ihre Grenzwerte überschreiten besser und schneller schützen, da der Wimpernschlag mit nur in 0.25 Sek. ist und in diesem energiereichen Bereich zu langsam ist. Wichtig ist das die Schutzvorrichtung im Nah- unter einem Meter und im Fernbereich funktioniert.
Die heutigen Laserschutzbrillen decken nur bestimmte Wellenlängen ab. Die Frequenzen von Lasergeräten die aber auf dem Markt vorhanden sind, beginnend mit der UV Strahlung die ab 157nm, 193nm, 213nm, 222nm, 224.3nm, 248nm, 266nm, XeCI Eximer 308nm, He-Cd 325nm, 332.4nm, 347nm, 351nm, 355nm und dann geht es weiter mit dem sichtbaren Licht wo man Laser mit folgenden Wellenlängen wie 402nm, 432nm, 441.6nm, 488nm, 510.5nm, grüner Laser mit 532nm, 539.5nm, 543.5nm, gelben 578.2nm, 594.1nm, bis zum roten Laser 611.9nm, 632.8nm, 647.1nm, 694nm und dann geht es weiter mit Infrarot Bereich wie Nd.Yag 946nm, Nd.Yag 1064, 1047nm, 1079nm, 1152nm 1315nm, 1319nm, 1523nm 1540nm, 2001nm, 2008nm, 2079nm, 2090nm, 3391nm bis in Ferne IR 1mm.
Für die Dämmerung und Nachtbereich benötigt man somit mindestens einen Lichttransmissionsgrad von mehr als 75%. (Siehe Normierung ISO12312-1 oder EN 166.) Entwicklungen oder Laserschutz die mit LCD, OLED und andere Technologien funktionieren, können auch nicht im Abend und im Nachtbereich eingesetzt werden. Grund dazu ist wieder der Lichttransmissionsgrad der über 75% sein muss. Die LCD Technologie funktioniert mit Polarisationsfilter, da liegt der Lichttransmissionsgrad unter den 75% ca. 40%-50%.
Dieses intensive Licht kann vorübergehend die Piloten und Fahrer ablenken. Bei den betroffenen kann die Sicht kurzfristig beeinflusst, gestört oder blockiert werden. Kritischen Phasen des Fluges sind: Start, Ansatz-, Lande- und Notfallmanöver. Blockiert die Brille für einen Bruchteil unter einer Sekunde, dann kann der Betroffene auf den Vorfall eingreifen und handeln. Im anderen Fall hat er keine Sicht für kurze oder längere Zeitspanne da das Auge mit der Strahlung überlastet wurde. Weitere Sorgen sind potenzielle Augenverletzungen, die das gesamte Auge betreffen, dazu gehören Hornhaut, Bindehaut, Iris, Linse, Glaskörper, Makula sowie Netzhaut. Hinzu kommt am Abend oder nachts der Unterschied von dunkler Umgebung mit starkem Licht die sehr hoch sind, damit wird das Auge durch die extreme Situation weiter belastet. (Weitere Informationen über den Laserschutz findet man unter der BGI 5092 und können dort gelesen werden).
Hinzu kommt, dass Laserstrahlen je nach Distanz sich die Punktgrösse (Strahlendurchmesser) unterschiedlich ist. Bei 10000m kann die Punktgrösse ca. 10m, bei 2500m ca. 2.5m, bei, 1250 m ca. 1.25m haben. Bei nähren Distanzen wie 50m ca. 0.05m und bei 25m 0.025m und bei einer Distanz von 10m von nur 0.01m. Damit besteht die Gefahr in näheren Einsatzgebieten, wie bei der Polizei, Tram-, Busfahrer, Lokomotivführer, Autofahrer etc. der Fall ist, wo diese Distanz eher unter 50m und weniger als 10 m eine andere Ausgangslage als beim Fliegen vorzufinden ist. Wobei Helikopterpiloten auch diesen Nahenbereich haben können. Je näher der Laserstrahl ist, umso intensiver ist die Energie. Somit muss das Auge in allen Situationen nach und fern geschützt werden. Auch Brillen mit Entspiegelung haben einen Lichttransmissionsgrad der unter 60% liegt und somit den 75% Lichttransmissionsgrad nicht erreicht. Dies sind die internationalen Richtlinien gemäss ISO 12312-1 und ISO 12311.1 oder EN 166.
Nimmt man die einzelnen Farben trotzdem weg wie es einige Hersteller es tun um die spezifische Wellenlänge zu stoppen, dann findet eine Farbdiskriminierung statt und somit ist die Sicherheit in der Fliegerei oder im Verkehrs- Schiffsbereich gefährdet. Laserschutzbrillen unterliegen den Europäischen Richtlinien zur Persönlichen Schutzausrüstung (PSA-Richtlinie 89/686/EWG). Sie sind nicht für den Strassenverkehr zugelassen.
Gemäss Norm DIN EN 207 muss der Schutz mindestens 10s standhalten.
Zum Beispiel einen höheren Schutz gegen Säuren, Laugen oder giftigen bzw. reaktiven Gasen und Dämpfen. Auch die Sensoren können vor Schmutz geschützt werden und werden je nach Einsatzgebiet mit dem entsprechenden transparenten oder getönten Material, wie Quarz, Polymere wie Polykarbonat, Polyester, Copolyester, Cellulose Propionate, Azetate oder andere Polymere geschützt.
Die Konstruktion des Laserschutzes darf nicht nur von der vorderen Seite sein sondern auch rundherum muss das Auge geschützt sein. Es darf weder seitlich, noch von oben oder unten die Strahlung hinein kommen. Es ist wichtig ein sehr grosses Sichtfeld beizubehalten, damit ist die Sicherheit nicht darunter leidet. Deshalb sollte man mindestens 90° bis 180° oder mehr an Sichtfeld gewährleisten.

Die Haut ist im Allgemeinen weniger empfindlich gegenüber Laserstrahlen als das Auge. Die Wirkung von Laserstrahlen auf die Haut hängt sehr stark von der Intensität der Strahlung ab und es entstehen nicht nur Schäden auf der obersten Hautschicht, sondern auch die untere Hautschicht kann durch die hohe Intensität betroffen sein. Laserstrahlung mit hoher Intensität kann zu Verbrennungen, starker Blasenbildung und späteren Vernarbungen der Haut führen.

| **Europäische Kennzeichnung** | **Amerikanische Kennzeichnung** | **Typische Leistung in Milliwatt (mW)** | **Beispiele von Anwendungen** |
|---|---|---|---|
| Klasse 1 | Klasse I | < 0,4 mW | DVD-Player |
| Klasse 2 | Klasse II | < 1 mW | Laserpointer |
| Klasse 3R | Klasse IIIa | < 5 mW | Showlaser |
| Klasse 3B | Klasse IIIb | < 500 mW | Showlaser, medizinische / kosmetische Laser |
| Klasse 4 | Klasse IV | > 500 mW | Showlaser; medizinische / kosmetische Laser |

Weitere Informationen findet man in der Literatur die auf die Grenzwerte die in mW und auch in Zeiteinheit wiedergegeben werden. Diese Werte können über die Software angepasst werden.

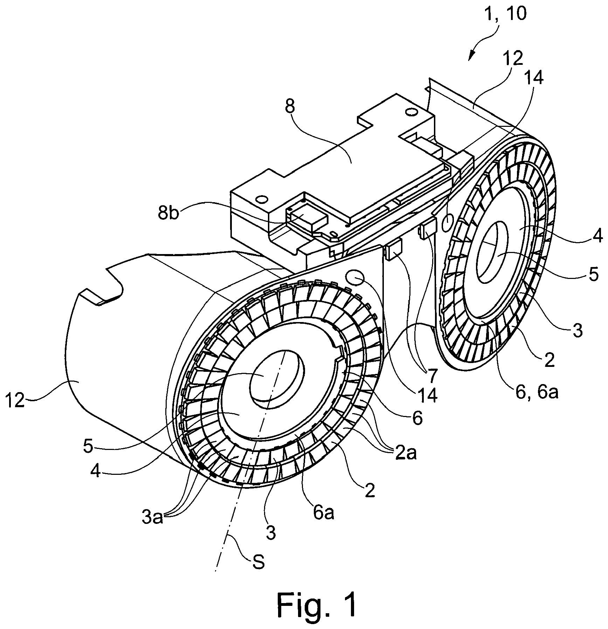

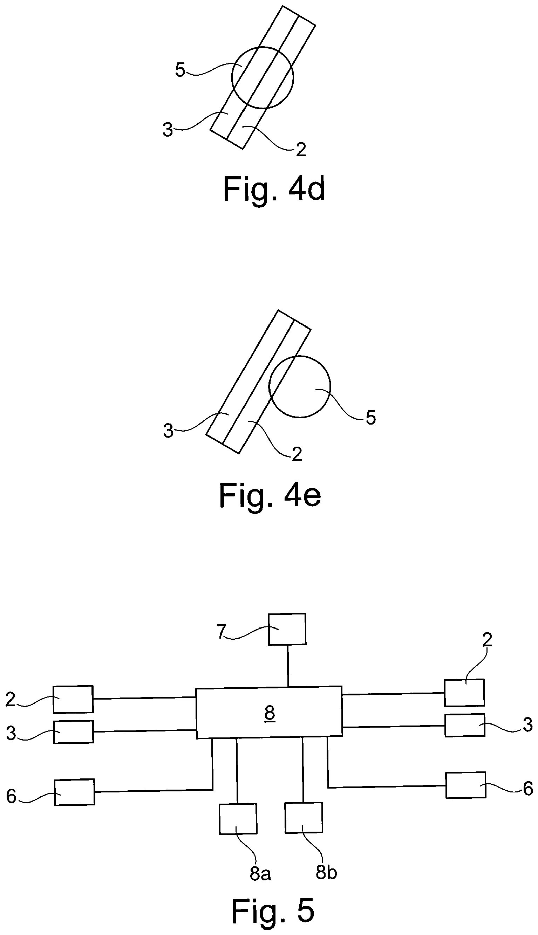

Fig. 1 zeigt eine als Brillenvorsatz ausgestaltete Vorrichtung 1 zum Schutz der Augen vor Strahlung, vorzugsweise im Bereich von 100nm bis 1 mm und insbesondere vor UV, Licht, oder IR. Die Vorrichtung 1 umfasst zumindest zwei Sensoranordnungen 2,3, eine äussere Sensoranordnung 2 sowie eine innere Sensoranordnung 3, wobei jede Sensoranordnung 2,3 eine Mehrzahl von nacheinander folgend angeordneten Strahlensensoren 2a,3a aufweist welche entlang je eines geschlossenen Kurvenzuges 2b, 3b angeordnet sind. Der Kurvenzug kann in einer Vielzahl möglicher Formen verlaufen, vorteilhafterweise kreisförmig oder oval, jedoch auch beispielsweise als Polygonzug, beispielsweise dreieckig oder viereckig. Die innere Sensoranordnung 3 ist von der äusseren Sensoranordnung 2 umschlossen, wobei die äussere und die innere Sensoranordnung 2,3 aneinander liegend angeordnet sind, und wobei die innere Sensoranordnung 3 einen Strahlendurchgangsbereich 4 umschliesst. Der Strahlendurchgangsbereich 4 weist eine Strahlendurchgangsöffnung 5 auf. Eine mechanische Schliessvorrichtung 6 ist derart bezüglich dem Strahlendurchgangsbereich 4 angeordnet, dass eine durch die Strahlendurchgangsöffnung 5 tretende Einfallsstrahlung S entweder durchgelassen oder zumindest teilweise abgeschwächt wird. Eine in Figur 5 dargestellte elektronische Ansteuervorrichtung 8 ist Signal leitend mit der inneren Sensoranordnung 2, der äusseren Sensoranordnung 3 sowie der Schliessvorrichtung 6 verbunden ist.

Die Schliessvorrichtung 6 umfasst zumindest ein mechanisch bewegliches Element 6a auf, welches die Einfallsstrahlung S entweder durchlässt oder zumindest teilweise abschwächt. Vorteilhafterweise lässt das bewegliche Element 6a den Strahl S entweder vollständig durch, oder unterbricht diesen vollständig. Die Schliessvorrichtung 6 weist vorzugsweise eine Verschlusszeit im Bereich von 10⁻³ bis 10⁻¹⁸ Sekunden auf.

Das mechanisch bewegliche Element 6a ist vorzugsweise strahlendicht oder in verschiedenen OD (Optical Density) ausgestaltet und kann insbesondere eine verspiegelte Oberfläche aufweisen.

Das mechanisch bewegliche Element 6a kann in einer vorteilhaften Ausgestaltung auch eine Strahlentransmission im Bereich von 5% bis 99% aufweisen.

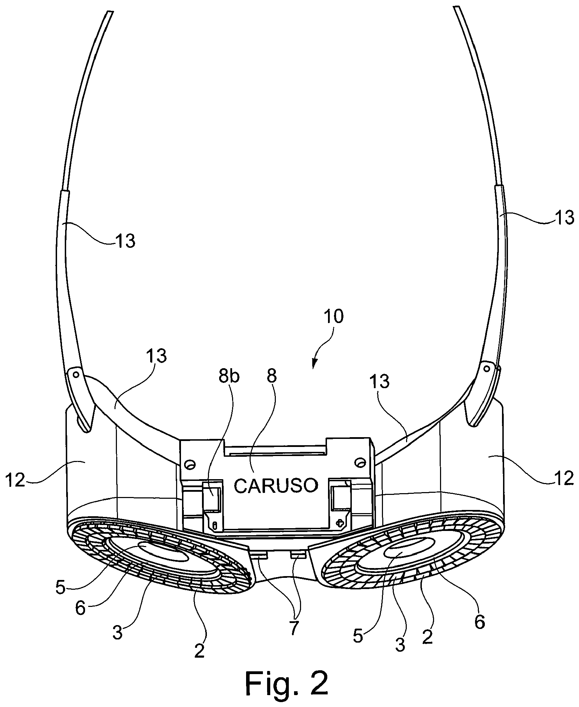

Die Vorrichtung 1 kann, wie in Figur 1 dargestellt, als Brille 10 oder wie in Figur 2 dargestellt als Brillenvorsatz 10 einer Brille 13 ausgestaltet sein. Die Brille 13 kann Korrekturgläser aufweisen. Die Brille 13 kann zudem ein Schutzfilter aufweisen, so dass diese eingefärbt ist und einen Lichttransmissionsgrad im Bereich von 100% bis 0% aufweist. Bereich Vorteilhafterweise weist die Vorrichtung 1 eine rundherum Abdeckung 12 auf, welche einen seitlichen Nichteintritt verhindert. Die Figuren 1 und 2 zeigen eine Vorrichtung 1 für Brillen oder als Brille, mit zwei gegenseitig beabstandet angeordneten Strahlendurchgangsbereichen 4 mit je einer Strahlendurchgangsöffnung 5 und je einer Schliessvorrichtung 6, wobei jeder Strahlendurchgangsbereich 4 von einer inneren und einer äusseren Sensoranordnung 2,3 umgeben ist.
Die beiden Schliessvorrichtungen 6 sind vorteilhafterweise individuell ansteuerbar.

Die in Figur 1 und 2 dargestellte Vorrichtung 1 umfasst vorteilhafterweise einen Umgebungsstrahlensensor 7 zur Erfassung der Umgebungsstrahlung.

Die Vorrichtung 1 könnte zudem ein nicht dargestelltes, lösbares optisches Filter umfassen, welches derart mit der Vorrichtung verbindbar ist, dass das optische Filter vor der Sensoranordnung 2,3, dem Strahlendurchgangsbereich 4 und vorzugsweise auch vor dem Umgebungsstrahlensensor 7 angeordnet ist, um eintreffende Strahlen zu filtern, um insbesondere den Umgebungsstrahlensensor 7 vor einer übermässigen Lichtintensität zu schützen.

Die Vorrichtung 1 weist wie in Figur 5 dargestellt eine Ansteuervorrichtung 8 und vorzugsweise eine Speichervorrichtung 8a sowie eine Datenschnittstelle 8b auf. Die Ansteuervorrichtung 8 ist signalleitend mit der äusseren Sensoranordnung 2 der inneren Sensoranordnung 3 sowie der Schliessvorrichtung 6 verbunden.

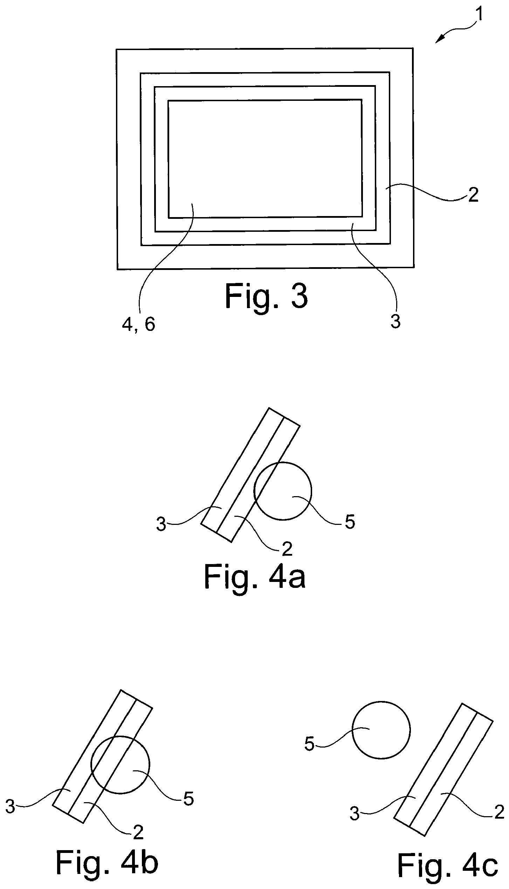

Figur 3 zeigt ein Ausführungsbeispiel einer Vorrichtung 1 mit äusserer und innerer Sensoranordnung 2,3, Strahlendurchgangsbereich 4 und Strahlendurchgangsöffnung 5. Die Strahlendurchgangsöffnung 5 ist gleichzeitig die Schliessvorrichtung 6, welche als elektrooptischer Strahlenverschluss ausgestaltet ist, beispielweise als LCD-Strahlenverschluss.

Die Vorrichtung wird derart betrieben, eine durch die Strahlendurchgangsöffnung 4 tretende Einfallsstrahlung S entweder durchgelassen oder zumindest teilweise und vorzugsweise abgeschwächt wird, wobei die Schliessvorrichtung 6 geschlossen wird, wenn die äussere Sensoranordnung 2 und nachfolgend die innere Sensoranordnung 3 von der Einfallsstrahlung S bestrahlt wird, und wobei die Schliessvorrichtung 6 wieder geöffnet wird, wenn vorerst die innere Sensoranordnung 3 und nachfolgend nur noch die äussere Sensoranordnung 2 von der Einfallsstrahlung S bestrahlt wird. Die Figuren 4a bis 4e zeigen solches Verfahren. Die Figuren zeigen abschnittweise eine äussere Sensoranordnung 2 sowie eine innere Sensoranordnung 3. In Figur 4a nähert sich der Einfallstrahl S der äusseren Sensoranordnung 2 und bestrahlt diese. In Figur 4b ist der Einfallstrahl S weiter fortgeschritten und bestrahlt zudem die innere Sensoranordnung 3. Sobald die Ansteuerungsvorrichtung 8 diesen Zustand erkennt wird die Schliessvorrichtung 6 geschlossen. Wie in Figur 4c dargestellt bewegt sich der Einfallstrahl S weiter in den Strahlendurchgangsbereich 4. Der Einfallstrahl S bewegt sich weiter und wird an irgendeiner Stelle aus dem Strahlendurchgangsbereich 4 wieder austreten, und dabei, wie in Figur 4d dargestellt, zuerst die innere Sensoranordnung 3 und danach die äussere Sensoranordnung 2 bis Strahlen. Der Einfallstrahl S bewegt sich weiter, und wird, wie in Figur 4e dargestellt, anschliessend die innere Sensoranordnung 3 nicht mehr bestrahlen und nur noch die äussere Sensoranordnung 2 bestrahlen. Sobald die Ansteuerungsvorrichtung 8 diesen Zustand erkennt wird die Schliessvorrichtung 6 wieder geöffnet. Dadurch ist sichergestellt, dass die Schliessvorrichtung 6 immer dann geschlossen ist, wenn sich der Einfallstrahl S innerhalb des Strahlendurchgangsbereichs 4 befindet.

Vorteilhafterweise ist für das linke als auch für das rechte Auge je eine Strahlendurchgangsöffnung 5 mit je einer Schliessvorrichtung 6 vorgesehen, wobei die Schliessvorrichtungen 6 vorteilhafterweise unabhängig voneinander von der jeweiligen Einfallsstrahlung S angesteuert werden.

Vorteilhafterweise wird mit dem Sensor 7 die Umgebungsstrahlung U gemessen und die Schliessvorrichtung 6 nur dann geschlossen und wieder geöffnet wird, wenn die von zumindest der äusseren Sensoranordnung 2 gemessene Strahlungsintensität der Einfallsstrahlung S höher ist als die Intensität der Umgebungsstrahlung U oder die Grenzwerte überschritten werden.

Vorteilhafterweise können die einzelnen Sensoren 2a, 3a, der äusseren Sensoranordnung 2 und vorzugsweise auch an der inneren Sensoranordnung 3 einzeln oder zumindest gruppenweise ausgelesen werden, sodass die Einfallsstrahlung S gemessen werden kann, und daraus eine Einfallsstrahlrichtung berechnet werden kann, weil bekannt ist welche der Sensoren 2a,3a durch die Einfallstrahlung S bestrahlt wurden. Nebst der Einfallsstrahlrichtung wird vorzugsweise auch eine Uhrzeit, eine GPS-Position, eine Höhe, Koordianten oder eine Strahlungsintensität der Einfallsstrahlung S gemessen und in einer Speichervorrichtung 8a gespeichert.

## Patentansprüche

1. Vorrichtung (1) zum Schutz der Augen vor Strahlung, vorzugsweise im Bereich von 100nm bis 1 mm und insbesondere vor UV, Licht, oder IR, umfassend zumindest zwei Sensoranordnungen (2,3), eine äussere Sensoranordnung (2) sowie eine innere Sensoranordnung (3), und wobei die äussere und die innere Sensoranordnung (2,3) aneinander liegend angeordnet sind, und wobei der Strahlendurchgangsbereich (4) eine Strahlendurchgangsöffnung (5) aufweist, und wobei eine Schliessvorrichtung (6) derart bezüglich dem Strahlendurchgangsbereich (4) angeordnet ist, dass eine durch die Strahlendurchgangsöffnung (5) tretende Einfallsstrahlung (S) entweder durchgelassen oder zumindest teilweise abgeschwächt wird, sowie umfassend eine Ansteuervorrichtung (8) welche mit der inneren Sensoranordnung (2), der äusseren Sensoranordnung (3) sowie der Schliessvorrichtung (6) verbunden ist, **dadurch gekennzeichnet dass**, jede Sensoranordnung (2,3) eine Mehrzahl von nacheinander folgend angeordneten Strahlensensoren (2a,3a) aufweist welche entlang je eines geschlossenen Kurvenzuges (2b,3b) angeordnet sind, dass die innere Sensoranordnung (3) von der äusseren Sensoranordnung (2)umschlossen ist, dass die innere Sensoranordnung (3) einen Strahlendurchgangsbereich (4) umschliesst.

2. Vorrichtung nach Anspruch 1, **dadurch gekennzeichnet, dass** die Schliessvorrichtung (6) zumindest ein mechanisch bewegliches Element (6a) aufweist, welches die Einfallsstrahlung (S) entweder durchlässt oder zumindest teilweise abschwächt, und dass die Schliessvorrichtung (6) eine Verschlusszeit im Bereich von 10⁻³ bis 10⁻¹⁸ Sekunden aufweist.

3. Vorrichtung nach Anspruch 2, **dadurch gekennzeichnet, dass** das mechanisch bewegliche Element (6a) strahlendicht ausgestaltet ist und insbesondere eine verspiegelte Oberfläche aufweist.

4. Vorrichtung nach Anspruch 2, **dadurch gekennzeichnet, dass** das mechanisch beweglich Element (6a) eine Strahlentransmission im Bereich von 5% bis 99% aufweist.

5. Vorrichtung nach Anspruch 1, **dadurch gekennzeichnet, dass** die Schliessvorrichtung (6) als elektrooptischer Strahlenverschluss ausgestaltet ist.

6. Vorrichtung nach einem der vorhergehenden Ansprüche, **dadurch gekennzeichnet, dass** diese als Brille (10) oder als Brillenvorsatz (10) ausgestaltet ist, mit zwei gegenseitig beabstandet angeordneten Strahlendurchgangsbereichen (4) mit je einer Strahlendurchgangsöffnung (5) und je einer Schliessvorrichtung (6), wobei jeder Strahlendurchgangsbereich (4) von einer inneren und einer äusseren Sensoranordnung (2,3) umgeben ist.

7. Vorrichtung nach Anspruch 6, **dadurch gekennzeichnet, dass** die beiden Schliessvorrichtungen (6) individuell ansteuerbar sind.

8. Vorrichtung nach einem der vorhergehenden Ansprüche, umfassend einen Umgebungsstrahlensensor (7) zur Erfassung der Umgebungsstrahlung, wobei der Umgebungsstrahlensensor (7) mit der Ansteuervorrichtung (8) verbunden ist.

9. Vorrichtung nach einem der vorhergehenden Ansprüche, umfassend ein lösbares optisches Filter (11), welcher derart mit der Vorrichtung verbindbar ist, dass das optische Filter (11) vor der Sensoranordnung (2,3), dem Strahlendurchgangsbereich (4) und vorzugsweise auch vor dem Umgebungsstrahlensensor (7) angeordnet ist.

10. Vorrichtung nach einem der vorhergehenden Ansprüche, wobei die Ansteuervorrichtung (8) eine Speichervorrichtung (8a) umfasst, in welchem zumindest die Einstrahlrichtung der Einfallsstrahlung (S) und vorzugsweise auch eine Uhrzeit, eine GPS-Position, Koordinaten oder eine Strahlungsintensität der Einfallsstrahlung (S) speicherbar ist oder weiter kommuniziert.

11. Verfahren zum Schutz der Augen vor Strahlung mittels einer Vorrichtung nach Anspruch 6, vorzugsweise im Bereich von 100nm bis 1 mm und insbesondere vor UV, Licht, oder IR, wobei das Auge von zumindest zwei Sensoranordnungen (2,3) umgeben ist, einer äusseren Sensoranordnung (2) sowie eine inneren Sensoranordnung (3), wobei jede Sensoranordnung (2,3) eine Mehrzahl von nacheinander folgend angeordneten Strahlensensoren (2a,3a) aufweist welche entlang je eines geschlossenen Kurvenzuges (2b,3b) angeordnet sind, wobei die die innere Sensoranordnung (3) von der äusseren Sensoranordnung (2) umschlossen ist, und wobei die innere Sensoranordnung (3) einen Strahlendurchgangsbereich (4) umschliesst, und wobei eine Strahlendurchgangsöffnung (5) im Strahlendurchgangsbereich (4) angeordnet ist, und wobei eine Schliessvorrichtung (6) derart angeordnet ist, dass eine durch die Strahlendurchgangsöffnung (4) tretende Einfallsstrahlung (S) entweder durchgelassen oder zumindest teilweise abgeschwächt wird, wobei die Schliessvorrichtung (6) geschlossen wird, wenn die äussere Sensoranordnung (2) und nachfolgend die innere Sensoranordnung (3) von der Einfallsstrahlung (S) bestrahlt wird, und wobei die Schliessvorrichtung (6) wieder geöffnet wird, wenn vorerst die innere Sensoranordnung (3) und nachfolgend nur noch die äussere Sensoranordnung (2) von der Einfallsstrahlung (S) bestrahlt wird.

12. Verfahren nach Anspruch 11, **dadurch gekennzeichnet, dass** sowohl für das linke als auch für das rechte Auge je eine Strahlendurchgangsöffnung (5) mit je einer Schliessvorrichtung (6) vorgesehen ist, und dass die Schliessvorrichtungen (6) unabhängig voneinander von der jeweiligen Einfallsstrahlung (S) angesteuert werden.

13. Verfahren nach Anspruch 11 oder 12, **dadurch gekennzeichnet, dass** eine Umgebungsstrahlung (U) gemessen wird, und dass die Schliessvorrichtung (6) nur dann geschlossen und wieder geöffnet wird, wenn die von zumindest der äusseren Sensoranordnung (2) gemessene Strahlungsintensität der Einfallsstrahlung (S) höher ist als die Intensität der Umgebungsstrahlung (U) oder die Grenzwerte überschritten werden.

14. Verfahren nach einem der Ansprüche 11 bis 13, **dadurch gekennzeichnet, dass** die Schliessvorrichtung (6) mit einer Verschlusszeit im Bereich von 10⁻³ bis 10⁻¹⁸ Sekunden geschlossen und/oder geöffnet wird.

15. Verfahren nach einem der Ansprüche 11 bis 14, **dadurch gekennzeichnet, dass** zumindest an der äusseren Sensoranordnung (2) und vorzugsweise auch an der inneren Sensoranordnung (3) der Bereich bestimmt wird, welcher von der Einfallsstrahlung (S) bestrahlt wird, und dass daraus eine Einfallsstrahlrichtung berechnet wird, und dass nebst der Einfallsstrahlrichtung vorzugsweise auch eine Uhrzeit, eine GPS-Position, Koordinaten oder eine Strahlungsintensität der Einfallsstrahlung (S) gespeichert wird oder weiter kommuniziert wird.

## Claims

1. A device (1) to protect the eyes from radiation, preferably in the range from 100 nm to 1 mm and in particular from UV, light, or IR, which is designed as glasses or spectacle attachment comprising of at least two sensor arrangements (2,3), an external sensor arrangement (2) and an internal sensor arrangement (3), and wherein the external and internal sensor arrangement (2,3) are arranged adjacently to each other, and wherein the radiation passage region (4) has a radiation passage opening (5), and wherein a closing device (6) is arranged relative to the radiation passage region (4) that an incident radiation (5) passing through the radiation passage opening (S) is either let through or is at least partially attenuated, and comprised of a control device (8) connected to the internal sensor arrangement (2), the external sensor arrangement (3) and the closing device (6), wherein each sensor arrangement (2,3) comprises a plurality of radiation sensors (2a,3a) arranged one after another along a closed curve (2b,3b) and wherein the internal sensor arrangement (3) is surrounded by the external sensor arrangement (2) and wherein the internal sensor arrangement (3) encloses a radiation passage region (4).

2. The device according to claim 1, **characterized in that** the closing device (6) has at least one mechanically movable element (6a) which lets the incident radiation (S) either pass or at least partially attenuate, and that the closing device (6) has a shutter speed in the range of 10 -3 to 10 -18 seconds.

3. The device according to claim 2, **characterized in that** the mechanically movable element (6a) is configured radiopaque and has a mirrored surface.

4. The device according to claim 2, **characterized in that** the mechanically movable element (6a) has a radiation transmission in the range of 5% to 99%.

5. The device according to claim 1, **characterized in that** the closing device (6) is designed as electro-optical radiation shutter.

6. The device according to one of the preceding claims, **characterized in that** it is designed as glasses (10) or as an spectacle attachment (10) with two reciprocally spaced-apart radiation passage regions (4) each having a radiation passage opening (5) and each a closing device (6), wherein each radiation passage region (4) is surrounded by an internal and an external sensor arrangement (2,3).

7. The device according to claim 6, **characterized in that** the two closing devices (6) are individually controllable.

8. The device according to one of the preceding claims, comprising of an ambient radiation sensor (7) for detecting the ambient radiation, wherein the ambient radiation sensor (7) is connected to the control device (8).

9. The device according to one of the preceding claims, comprising of a detachable optical filter (11), which can be connected in such a way with the device that the optical filter (11) is arranged in front of the sensor arrangement (2,3), the radiation passage region (4) and preferably also in front of the ambient radiation sensor (7).

10. The device according to one of the preceding claims wherein the control device (8) comprises of a storage device (8a), in which at least the direction of incidence of the incident radiation (S) and preferably also a time stamp, GPS position, coordinates or radiation intensity of the incident radiation (S) can be stored or further communicated.

11. A method for protecting the eyes from radiation by means of a device as described under claim 6, preferably in the range from 100 nm to 1 mm and in particular from UV, light, or IR, where the eye is surrounded by at least two sensor arrangements (2,3), an external sensor arrangement (2) and an internal sensor arrangement (3), wherein each sensor arrangement (2,3) is arranged in a plurality of sequentially followed radiation sensors (2a, 3a) disposed along a closed curve path (2b, 3b), wherein the internal sensor arrangement (3) is surrounded by the external sensor arrangement (2), and wherein the internal sensor arrangement (3) encloses a radiation passage region (4), and wherein a radiation passage opening (5) is arranged in the radiation passage region (4), and wherein a closing device (6) is arranged in such a way with respect to the radiation passage opening that an incident radiation (S)will either pass or at least partially attenuate, and that the closing device (6) is closed if the external sensor arrangement (2) and subsequently the internal sensor arrangement (3) is irradiated by the incident radiation (S), and wherein the closing device (6) is re-opened if initially the internal sensor arrangement (3) and subsequently only the external sensor arrangement (2) is irradiated by the incident radiation (S).

12. The method according to claim 11, **characterized in that** for both, the left and for the right eye each, a radiation passage opening (5) each having a closing device (6) is planned, and that the closing devices (6) are controlled independently of the respective incident radiation (S).

13. The method according to claim 11 or 12, **characterized in that** an ambient radiation (U) is measured, and that the closing device (6) is only closed and re-opened if the radiation intensity of the incident radiation (S), measured by the external sensor arrangement (2) at least, is higher than the intensity of the ambient radiation (U) or if the thresholds are exceeded.

14. The method according to one of the claims 11 to 13, **characterized in that** the closing device (6) can open or close with a shutter speed in the range of 10⁻³ to 10⁻¹⁸ seconds.

15. The method according to one of the claims 11 to 14, **characterized in that** at least on the external sensor arrangement (2) and preferably also on the internal sensor arrangement (3) the area can be determined in which is irradiated by the incident radiation (S), and that therefrom the incident radiation direction can be calculated, and that in addition to the incident radiation direction preferably also a time stamp, GPS position, coordinates or radiation intensity of the incident radiation (S) can be stored or can be further communicated.

## Revendications

1. Cet appareil (1) est conçu pour protéger vos yeux des radiations dont les longueurs d'ondes sont de préférence comprises entre 100 nm et 1 mm, et en particulier des ultraviolets, de la lumière et des infrarouge qui est conçu comme des lunettes ou une monture de lunettes, il est équipé d'aux moins deux dispositifs de capteurs (2,3) : un dispositif de capteurs externes (2) et un dispositif de capteurs internes (3) et ces derniers (2,3) se situent l'un à côté de l'autre et la zone de passage des radiations (4) comporte une ouverture pour les radiations (5), et le système de fermeture (6) se trouve près de la zone de passage des radiations (4), afin de laisser passer ou d'atténuer en partie la radiation incidente (5) qui passe par la zone de passage des radiations (S). L'appareil comprend également un dispositif de contrôle (8) qui est connecté au dispositif de capteurs internes (2), au dispositif de capteurs externes (3) et au système de fermeture (6), et caractérisé que chaque dispositif de capteurs (2,3) possède une multitude de capteurs de radiations (2a,3a) situés les uns derrières les autres le long d'une courbe fermée (2b,3b), le dispositif de capteurs externes (2) entoure le dispositif de capteurs internes (3), le dispositif de capteurs internes (3) entoure la zone de passage des radiations (4).

2. La revendication 1 indique que le système de fermeture (6) dispose d'au moins un élément mécaniquement mobile (6a) qui permet de laisser passer ou d'atténuer en partie la radiation incidente (S) et que la vitesse d'obturation du système de fermeture (6) est comprise entre 10 -3 to 10 -18 secondes.

3. La revendication 2 indique que l'élément mécaniquement mobile (6a) est radio-opaque et qu'il dispose d'une surface réfléchissante.

4. La revendication 2 indique que la transmission des radiations de l'élément mécaniquement mobile (6a) est comprise entre 5% to 99%.

5. La revendication 1 indique que le système de fermeture (6) est conçu comme obturateur de radiations électro-optique.

6. L'une des précédentes revendications indique que l'appareil a été conçu comme une paire de lunettes (10) ou une monture de lunettes (10). Il comporte deux zones de passage des radiations (4) distinctes possédant chacune une ouverture pour le passage des radiations (5) et un système de fermeture (6), chaque zone de passage des radiations (4) est entourée par le dispositif de capteurs internes et le dispositif de capteurs externes (2,3).

7. La revendication 6 indique que les deux systèmes de fermeture (6) peuvent être contrôlés séparément.

8. L'une des revendications précédentes indique que l'appareil est équipé d'un capteur de radiations ambiantes (7) permettant de détecter la radiation ambiante. Le capteur de radiations ambiantes (7) est connecté au dispositif de contrôle (8).

9. L'une des revendications précédentes indiquent que l'appareil comporte un filtre optique détachable (11), que l'on peut connecter à l'appareil de manière que le filtre optique (11) se trouve devant les dispositifs de capteurs (2,3), la zone de passage des radiations (4) et de préférence également devant le capteur de radiations ambiantes (7).

10. L'une des revendications précédentes indiquent le dispositif de contrôle (8) comporte un dispositive de stockage (8a) grâce auquel il est possible de stocker et d'indiquer la direction de la radiation incidente (S) l'estampille temporelle, la position GPS, les coordonnées et l'intensité du de la radiation incidente (S).

11. Comme l'indique la revendication 6, cet appareil permet de protéger vos yeux des radiations, dont les longueurs d'ondes sont de préférence comprises entre 100 nm et 1 mm, et en particulier des ultraviolets, de la lumière et des infrarouges. L'oeil est entouré par deux dispositifs de capteurs (2,3): un dispositif de capteurs externes (2) et un dispositif de capteurs internes (3). Chaque dispositif de capteurs est constitué d'une multitude de capteurs de radiations (2a, 3a), situés les uns derrières les autres le long d'une courbe fermée (2b, 3b). Le dispositif de capteurs externes (2) entoure le dispositif de capteurs internes (3). Le dispositif de capteurs internes (3) entoure la zone de passage des radiations (4). L'ouverture de la zone de passage des radiations (5) se situe dans la zone de passage des radiations (4). Le système de fermeture (6), est disposé de manière que l'ouverture de la zone de passage des radiations laisse passer ou atténue en partie la radiation incidente (S). Le système de fermeture (6) se ferme si la radiation incidente (S) irradie le dispositif de capteurs externes (2) et le dispositif de capteurs internes (3). Le système de fermeture (S) s'ouvre à nouveau si seul le dispositif de capteurs internes (3) ou si seul le dispositif de capteurs externes (2) est irradié par la radiation incidente.

12. La revendication 11 indique que la méthode mise en place prévoit que l'oeil droit et l'oeil gauche disposent tous deux d'une zone de passage des radiations (5), elles-mêmes équipées d'un système de fermeture (6). Les systèmes de fermeture (6) sont contrôlés indépendamment de leur radiation d'incidence (S) respective.

13. Les revendications 11 et 12 indiquent que la radiation ambiante (U) est mesurée et que le système de fermeture (6) s'ouvre et se ferme uniquement si l'intensité de de la radiation incidente (S), mesurée par le dispositif de capteurs externes (2) est plus élevée que l'intensité de la radiation ambiante (U) ou si le seuil est dépassé.

14. Les revendications 11 à 13 indiquent que le système de fermeture (6) s'ouvre et se ferme si la vitesse d'obstruction est comprise entre 10⁻³ to 10⁻¹⁸ secondes.

15. Les revendications 11 à 14 indiquent qu'il est possible de déterminer la zone irradiée par la radiation d'incidence (S) sur le dispositif de capteurs externes (2) et de préférence sur le dispositif de capteurs internes (3) également. Ainsi, il est possible de calculer la direction de la radiation d'incidence. De plus, on peut stocker et communiquer l'estampille temporelle, la position GPS, les coordonnées et l'intensité de la radiation d'incidence (S).
